# EUROPÄISCHE PATENTSCHRIFT

(11) **EP 1 130 084 B1**
(45) Veröffentlichungstag und Bekanntmachung des Hinweises auf die Patenterteilung: **04.08.2004**
(21) Anmeldenummer: 01105083.8
(22) Anmeldetag: 02.03.2001
(51) Int. Cl.: C12M 1/107, B01F 7/00

(54) **Biogasanlage zur Fermentation von organischen Stoffen**
Biogas plant for the fermentation of organic material
Installation de biogaz pour la fermentation de matériaux organiques

(30) Priorität: 04.03.2000 DE 10010672
(43) Veröffentlichungstag der Anmeldung: 05.09.2001
(73) Patentinhaber: U.T.S. Umwelt-Technik-Süd GmbH, 84419 Obertaufkirchen (DE)
(72) Erfinder: Bürger, Adam, 84405 Grüntegernbach (DE)
(74) Vertreter: Neubauer, Hans-Jürgen, Dipl.-Phys.

(56) Entgegenhaltungen:
- EP-A- 0 051 941
- EP-A- 0 144 213
- EP-A- 0 638 371
- WO-A-84/03126
- WO-A-93/19836
- DE-A- 2 419 305
- DE-C- 19 732 198

## Beschreibung

Die Erfindung betrifft eine Biogasanlage zur Fermentation von organischen Stoffen nach dem Oberbegriff des Anspruchs 1.

In derartigen Biogasanlagen läuft ein Fermentationsprozess ab, bei dem organische Stoffe wie z. B. Gras, Stalldung, Jauche, Klärschlamm, Stroh und dergleichen vergast werden. Dieser Fermentationsprozess findet in einem Fermenterbehälter statt, in dem die organischen Stoffe mit Flüssigkeit versetzt werden und der Fermentations- bzw. Vergasungsprozess unter aeroben oder anaeroben Bedingungen durch Mikroorganismen wie z. B. Hefen, Bakterien, etc. durchgeführt wird. Die bei der Fermentation entstehenden Biogase sammeln sich in einem oberen Fermenterbehälterbereich und können direkt zur Energieerzeugung verwendet werden, z. B. als Heizgas zur Stromerzeugung in nachgeschalteten Brennkraftmaschinen mit Elektrogeneratoren. Solche Biogasanlagen zur Fermentation von organischen Stoffen finden meist in der dezentralen Verarbeitung und Verwertung von organischen Reststoffen im landwirtschaftlichen Bereich Verwendung. Landwirte sind damit in der Lage nicht nur Nahrungsmittel zu produzieren, sondern durch Verwertung von organischen Reststoffen Energie zu erzeugen. Damit werden Stoffkreisläufe geschlossen und überfällige organische Reststoffe werden zudem nach der Vergärung zu einem hochwertigen Düngemittel, das wiederum auf die Pflanzen ausgebracht werden kann.

Für eine gleichmäßige Verteilung der Feststoffe in der Schlämmung und für das Verhindern des Festsetzens der Feststoffe an den Fermenterseitenwänden ist ein Rührwerk als Umwälzeinrichtung im Fermenterbehälter erforderlich.

Bei einer bekannten gattungsgemäßen Biogasanlage (DE 197 32 198 C1) ist im Fermenterbehälter ein Tauchmotor-Rührgerät angeordnet. Als Tauchmotor wird hier wie in allen bisherigen Biogasanlagen ein Elektromotor verwendet. Zu einer Energiezufuhr wird ein Elektrokabel verwendet, das von einer Energiequelle außerhalb des Fermenterbehälters dicht durch eine Behälterwand und weiter durch den Gasbereich zum Tauchmotor des Tauchmotor-Rührgeräts geführt ist. Mit einem solchen Tauchmotor-Rührgerät ist eine wirtschaftliche und energiesparende Homogenisierung im Fermenterbehälter möglich. Dazu ist das Tauchmotor-Rührgerät an einer Hubvorrichtung vertikal und horizontal verstellbar angeordnet, so dass das Tauchmotor-Rührgerät in einer optimalen Position arbeiten kann.

Zudem ist hier ein Service-Dom über einer Rührwerköffnung in einer Fermenterbehälter-Deckenwand vorgesehen, in den ein Tauchmotor-Rührgerät hochziehbar und gefahrlos für Servicepersonen gewartet und überprüft werden kann.

Für die vertikale und/oder horizontale Verstellung sind Handantriebe oder Elektroantriebe gezeigt, die zum Teil im Fermenterbehälterbereich angeordnet und ähnlich wie das Tauchmotor-Rührgerät über Elektrokabel angeschlossen sind. Insbesondere ist eine elektrische Seilwinde zum Hochziehen des Tauchmotor-Rührgeräts verwendet.

Biogase im Gasbereich des Fermenterbehälters sind explosible Gase, so dass dieser Gasbereich und gegebenenfalls ein Zonenbereich außerhalb des Fermenters grundsätzlich als Explosionsschutzbereich anzusehen sind. Da elektrisch betriebene Tauchmotor-Rührgeräte nur im eingetauchten Zustand, das heißt im Flüssigkeitsbereich arbeiten, sind diese nicht unmittelbar in einem Explosionsschutzbereich angeordnet. Daher werden derzeit keine explosionsgeschützten Tauchmotor-Rührgeräte in Fermenterbehältern von Biogasanlagen verwendet, da explosionsgeschützte Tauchmotoren gegenüber nicht explosionsgeschützten Geräten erheblich teurer sind. Durch Leckagen, Fehlbedienungen, etc. kann jedoch nicht ausgeschlossen werden, dass Tauchmotor-Rührgeräte auch im eingeschalteten Zustand in Verbindung mit dem Explosionsschutzbereich gelangen, so dass sich Gefahrzustände ergeben können. Die derzeitigen deutschen Richtlinien und Gesetze enthalten jedoch keine konkreten, auf Biogasanlagen anwendbaren Aussagen zum Explosionsschutz.

Zudem ergeben sich in der Praxis immer wieder elektrische Probleme wegen undichter elektrischer Tauchmotoren von Tauchmotor-Rührgeräten mit Gefahren für den Personenschutz und mit erforderlichen aufwendigen Wartungs- und Reparaturarbeiten.

Bekannte handbetätigbare Handseilwinden zum Anheben und Absenken von Tauchmotor-Rührgeräten erfordern eine körperlich harte Arbeit. Bei elektrisch betriebenen Seilwinden mit außerhalb des Fermenterbehälters angeordnetem Elektromotor ist eine aufwendige, wartungsintensive und anfällige gasdichte Durchführung des Seils durch eine Behälterwand notwendig. Eine elektrische Seilwinde gemäß DE 197 32 198 C1, Fig. 1 ist mit ihrem Elektromotor ständig im explosiblen Gasbereich angeordnet mit den nur aufwendig zu lösenden Problemen eines Explosionsschutzes.

Aufgabe der Erfindung ist es, eine gattungsgemäße Biogasanlage betriebssicherer und einfacher bedienbar zu gestalten.

Diese Aufgabe wird mit den Merkmalen des Anspruchs 1 gelöst.

Gemäß Anspruch 1 ist der Tauchmotor ein durch ein Arbeitsmedium betreibbarer Motor. Die Energie-Leitung umfasst einerseits eine Rohr- oder Schlauchleitung als Zuführleitung für das Medium die an eine Medium-Kompressionseinheit angeschlossen ist. Andererseits umfasst die Energie-Leitung eine Rohr- oder Schlauchleitung als Abführleitung für das Medium. Die Abführleitung ist vom Tauchmotor durch den Gasbereich und dicht durch eine Behälterwand aus dem Fermenterbehälter heraus geführt. Die Medium-Kompressionseinheit ist elektrisch betreibbar und außerhalb des Fermenterbehälters angeordnet.

Gemäß Anspruch 2 ist der Tauchmotor ein Hydraulik-Motor. Das Arbeitsmedium ist eine Hydraulikflüssigkeit und die Kompressionseinheit ist ein Hydraulik-Kompressor. Die Abführleitung ist dabei als Rückleitung in einem geschlossenen Kreislauf an den Hydraulik-Kompressor angeschlossen.

Alternativ dazu kann nach Anspruch 3 der Tauchmotor ein Pneumatik-Motor sein, wobei als Arbeitsmedium Luft verwendet ist und die Kompressoreinheit als Luftkompressor ausgebildet ist. Bei dieser Anordnung reicht es aus, wenn die Abführleitung als Abblasleitung lediglich dicht aus dem Fermenterbehälter geführt ist. Bei Verwendung eines anderen Gases, beispielsweise eines Inertgases kann auch hier ein geschlossener Gaskreislauf mit einer Gasrückführung zum Luftkompressor eingesetzt werden.

Sowohl in der Hydraulik- als auch in der Pneumatikausführung ergeben sich die Vorteile, dass die elektrisch betriebenen Kompressoren außerhalb des Fermenterbehälters angeordnet werden können und im Fermenterbehälter der Explosionsschutz durch die Verwendung von Hydraulik- oder Gasleitungen und die entsprechenden Hydraulik- oder Gasmotoren einfach beherrschbar ist. Zudem können keine elektrischen Probleme mit Tauchmotoren auftreten.

Gegenüber der bisherigen elektrischen Energieversorgung mit Elektrokabeln ist erfindungsgemäß zumindest im Fermenterbehälter ein Zwei-Leitungssystem mit Rohr- oder Schlauchleitungen als Medium-Zuführleitung und Medium-Abführleitung erforderlich. Insbesondere bei Verwendung eines Pneumatik-Motors als Tauchmotor ist ein sonst übliches Abblasen von Luft unmittelbar am Motor nicht möglich, da diese Luft in den Flüssigkeitsbereich und dann in den dichten Gasbereich gelangen würde und dort zu einer unerwünschten Verdünnung des Biogases und einer Gasvermehrung führen würde.

Mit der erfindungsgemäßen Energieversorgungstechnik sind auch weitere Antriebe im Fermenterbehälter ohne Explosionsschutzprobleme einfach automatisierbar, wie beispielsweise eine Seilwinde im Fermenterbehälter, so dass ein größeres Automatisierungspotential geschaffen wird.

Die Leitungen können insgesamt relativ dünn gehalten werden, so dass eine platzgünstige und einfache Installation möglich ist.

Nach Anspruch 4 können weitere Hydraulik-Motoren und/oder Pneumatik-Motoren der Biogasanlage mittels nur einer zentralen Kompressionseinheit betrieben werden. Solche Motoren können Pumpenmotoren und/oder Bodenräumgeräte-Motoren und/oder Schutzvorrichtungsmotoren und/oder Schiebermotoren für Rohrleitungen und/oder Hubmotoren für Tauchmotor-Rührgeräte und/oder Schwenkmotoren für Tauchmotor-Rührgeräte sein. Explosionsschutzprobleme sind damit in jedem Fall ausgeschlossen. Hohe Standzeiten mit langen Wartungsintervallen sind realisierbar.

Die einzelnen Motoren sind einfach über an sich bekannte Ausführungsformen von Steuereinheiten, die im Wesentlichen aus Magnetventilen aufgebaut sein können, ansteuerbar. Ansteuerungen können beispielweise individuell von Hand, zeitgesteuert, programmgesteuert oder innerhalb von Regelkreisen erfolgen. Damit sind Biogasanlagen bei einem kompakten und kostengünstigen Aufbau der Antriebs- und Steuersysteme hoch automatisierbar und professionell bedienbar.

Die automatisierte Bedienung verhindert harte körperliche Bedienarbeit an den Geräten der Biogasanlage. Die Geräte können zudem stufenlos während der Arbeit an den jeweiligen Leistungsbedarf z. B. durch eine Drehzahlregulierung angepasst werden, wobei insbesondere eine Anpassung an unterschiedliche Feststoffanteile, dickere oder dünnere Flüssigkeiten, mehr oder weniger Füllstand, etc. möglich ist.

Nach Anspruch 5 ist in an sich bekannter Weise das Tauchmotor-Rührgerät an einer Hubvorrichtung vertikal verstellbar angeordnet. Die Hubvorrichtung umfasst einen vertikal im Fermenterbehälter angebrachten stützenförmigen Rührwerkträger an dem das Tauchmotor-Rührgerät verschiebbar gehalten ist. Im oberen Bereich des Rührwerkträgers ist als Bestandteil einer betätigbaren Seilwinde eine Seiltrommel mit einer Seilverbindung durch ein aufwickelbares Seil zum Tauchmotor-Rührgerät angeordnet. Eine solche Anordnung eignet sich besonders für einen hydraulischen oder pneumatischen Antrieb des Tauchmotor-Rührgeräts.

In einer ersten Alternative ist die Energie-Leitung, bestehend aus der Zuführleitung und der Abführleitung im oberen Bereich des Rührwerkträgers festgelegt und in losen Schlaufen um das Seil zum Tauchmotor-Rührgerät gewickelt. Die Schlaufen sind dann je nach Verstellposition des Tauchmotor-Rührgeräts mehr oder weniger zusammengeschoben, so dass eine Festlegung der Energie-Leitung am Seil jedoch keine mechanische Überbeanspruchung der Energie-Leitung erfolgt.

In einer anderen Alternative nach Anspruch 7 ist für die Energie-Leitung mit Zuführleitung und Abführleitung eine betätigbare Trommel als Energie-Leitungs-Trommel vorgesehen, auf der entsprechend und zugeordnet dem Seilwicklungsstand die Energie-Leitung aufwickelbar ist. Die Einstellung des Wicklungsgrades der Energie-Leitung soll dabei so getroffen werden, dass in jeder Verstellposition des Tauchmotor-Rührgeräts nur ein Zug auf das Seil wirkt, die Energie-Leitung jedoch ohne größeren mechanischen Zug lediglich entsprechend ab- oder aufgewickelt wird.

Ein Problem ergibt sich daraus, dass die Energie-Leitung dicht und unverdrehbar durch eine Fermenterbehälterwand nach außen geführt ist, bei einer Trommeldrehung jedoch verdreht wird. Dieses Problem wird dadurch gelöst, dass die Energie-Leitungs-Trommel eine axiale Drehkopplung aufweist mit einem nichtdrehenden Kupplungsteil an den der Teil der Energie-Leitung zum ortsfesten Behälterwanddurchführung angeschlossen ist. Am anderen dagegen drehbaren Kupplungsteil ist der Teil der Energie-Leitung angeschlossen, der auf der Energie-Leitungs-Trommel aufwickelbar und abwickelbar ist.

Zweckmäßig werden nach Anspruch 8 die Seiltrommel und die Energie-Leitungs-Trommel gleichachsig und drehbewegungsübertragend gekoppelt, so dass dafür insgesamt nur ein Antrieb erforderlich ist.

In einer besonders einfachen und kompakten Ausführungsform nach Anspruch 9 wird vorgeschlagen, dass die Seiltrommel zugleich auch die Energie-Leitungs-Trommel ist, wobei die Energie-Leitung und das Seil über ihre Länge verbunden sind, beispielsweise durch beabstandete Schellen oder Klippverbindungen. Eine besonders innige Verbindung ergibt sich nach Anspruch 10 dadurch, dass das Seil aus einem Stahlgeflecht gebildet ist, das die darin integrierte Zuführleitung und/oder Abführleitung der Energie-Leitung umgibt. Das umgebende Stahlgeflecht übernimmt hier die Zugfunktion während die darin integrierten Hohlleitungen für den Medienfluss verwendet sind.

In einer anderen Ausführungsform nach Anspruch 11 sind das Seil und/oder die Energie-Leitung in einem aus mehreren Teleskoprohren bestehenden Teleskoprohr geführt. Das Teleskoprohr erstreckt sich vertikal entlang des Rührwerkträgers. Das obere Teleskoprohr ist dabei in einem oberen Bereich des Fermenterbehälters festgelegt und das untere Teleskoprohr ist unmittelbar oder mittelbar mit dem Tauchmotor-Rührgerät verbunden. Damit wird das Teleskoprohr zusammen mit einer Bewegung des Tauchmotor-Rührgeräts selbsttätig ausgezogen oder zusammengezogen. Das Seil und/oder die Energie-Leitung sind dabei gegen ungünstige Umgebungseinflüsse durch die Behälterflüssigkeit, insbesondere gegen darin enthaltene Feststoffe geschützt.

Ein solches Teleskoprohr kann nach Anspruch 12 im Rührwerkträger oder nach Anspruch 13 den Rührwerkträger umgebend angebracht sein.

In einer weiteren alternativen und besonders bevorzugten Ausführungsform ist nach Anspruch 14 vorgesehen, dass das Tauchmotor-Rührgerät an einem Stülprohr, vorzugsweise an einem unteren Stülprohrende, angeordnet ist. Dieses Stülprohr ist teleskopartig, vorzugsweise mit einem Spaltabstand, über einen vertikalen Behälter angebrachten, stützenförmigen Rührwerkträger stülpbar und daran in Rührwerkträgerlängsrichtung zur Einstellung unterschiedlicher Tauchmotor-Höhenpositionen im Fermenterbehälter verschiebbar geführt. Wieter ist das Stülprohr in jeder Tauchmotor-Höhenposition mit wenigstens einem Teilbereich des Stülprohrs dicht durch den Fermenterbehälter geführt. Die Energieleitung ist in einem derartigen Fall vom Tauchmotor kommend gasdicht im Stülprohr und/oder im Rührwerkträger nach oben aus dem Fermenterbehälter herausgeführt.

Bei einem derartigen Aufbau kann auf eine im Fermenterbehälter angeordnete Seilwinde verzichtet werden, da es für eine Höhenverstellung des Tauchmotors ausreicht, dass das mit einem Ende aus der Fermenterbehälterwand herausgeführte Stülprohr von außen angehoben oder abgesenkt wird. Eine gasdichte Durchführung kann beispielsweise mittels einer herkömmlichen Stopfbuchsendichtung auf einfache und preiswerte Weise hergestellt werden und/oder durch eine einfache Fettschmierung. Durch den Spaltabstand zwischen dem Stülprohr und dem Rührwerkträger wird ferner eine einfache Verschiebbarkeit des Stülprohrs relativ zum Rührwerkträger ermöglicht.

In einer nach Anspruch 15 besonders bevorzugten Ausgestaltung ist vorgesehen, dass das Stülprohr und damit der Tauchmotor mit einer außerhalb des Fermenterbehälters anordenbaren Hubvorrichtung anhebbar oder absenkbar ist. Eine derartige Hubvorrichtung ist beispielsweise eine ortsfeste Seilwinde, deren Seil über einen in etwa wenigstens auf maximaler Stülprohr-Hubhöhe angeordneten, ortsfesten Umlenkpunkt zu einer am oberen Stülprohrende angeordneten Seilanbindungsstelle geführt ist. Als Umlenkpunkt kann hier beispielsweise eine ortsfeste Umlenkrolle dienen. Eine derartige Seilwinde kann beispielsweise von Hand bedient werden, zum Beispiel mittels einer Kurbel, die dann in einer entsprechenden Stellung festlegbar ist. Alternativ dazu kann der Antrieb aber auch motorisch, zum Beispiel über einen Elektromotor erfolgen, wobei hier über den Elektromotor dann eine vorteilhafte Selbsthemmung möglich ist, die das Stülprohr und damit den Tauchmotor in einer bestimmten Höhenstellung hält. Anstelle einer derartigen Höhenverstellung mittels einer Seilwinde kann aber auch vorgesehen sein, dass das Anheben oder Absenken des Stülprohres mittels einer Ritzel-Zahnstangen-Anordnung erfolgt, wobei beispielsweise die Zahnstange an einem oberen Stülprohrende ausgebildet ist, der ein zum Beispiel über einen Motor antreibbares Ritzel zugeordnet ist.

In einer weiteren Ausgestaltung ist gemäß Anspruch 16 vorgesehen, dass das Stülprohr einen geschlossenen Profilquerschnitt und der Rührwerk-Träger einen zum Tauchmotor hin ausgerichteten, offenen Profilquerschnitt aufweist. Es sind dabei sowohl runde als auch rechteckförmige Querschnitte möglich. Insbesondere kann bei einem derartigen Aufbau das Stülprohr oben auch offen sein, da sich im Inneren des Stülprohrs und des Rührwerkträgers ein dem umgebenden Flüssigkeitspegel entsprechender Flüssigkeitsstand einstellt, der für eine Abdichtung gegen den Gasbereich sorgt.

Eine besonders einfache und servicefreundliche Anordnung ergibt sich in Verbindung mit einem an sich bekannten Service-Dom über einer Rührwerköffnung nach Anspruch 17.

Anhand einer Zeichnung wird die Erfindung näher erläutert.

Es zeigen:
- Fig. 1: ein schematischer Querschnitt durch eine Biogasanlage mit einem hydraulisch oder pneumatisch durch ein Arbeitsmedium betreibbaren Tauchmotor, einer hydraulisch oder pneumatisch betreibbaren Seiltrommel, sowie einem hydraulisch oder pneumatisch betreibbaren Rührwerkträger-Schwenkmotor,
- Fig. 2: eine schematische Schnittansicht entlang der Linie A-A der Fig. 1,
- Fig. 3: ein schematischer Querschnitt durch eine Biogasanlage mit einem hydraulisch oder pneumatisch durch ein Arbeitsmedium betreibbaren Tauchmotor, wobei eine Energieleitung in losen Schlaufen um ein Seiltrommel-Seil gewickelt ist,
- Fig. 4: ein schematischer Querschnitt durch eine Biogasanlage mit einem hydraulisch oder pneumatisch durch ein Arbeitsmedium betreibbaren Tauchmotor, wobei zusätzlich eine Energie-Leitungs-Trommel vorgesehen ist,
- Fig. 5: ein schematischer Querschnitt durch eine Biogasanlage mit einem hydraulisch oder pneumatisch durch ein Arbeitsmedium betreibbaren Tauchmotor sowie mit einem sich vertikal im Inneren eines Rührwerkträgers erstreckenden Teleskoprohr, bei dem die Energie-Leitung des Tauchmotors innerhalb des Teleskoprohrs verläuft,
- Fig. 6: ein schematischer Querschnitt durch eine Biogasanlage mit einer alternativen Ausführungsform eines Teleskoprohrs, bei der die Energie-Leitung zwischen einem Rührwerkträger und einer Teleskoprohrinnenwand geführt ist,
- Fig. 7: ein schematischer Querschnitt durch eine Biogasanlage mit einem hydraulisch oder pneumatisch durch ein Arbeitsmedium betreibbaren Tauchmotor sowie mit einer teleskopartigen Stülprohr-Rührwerkträger-Anordnung,
- Fig. 8: ein schematischer Querschnitt zweier alternativer Ausführungsformen entlang der Linie B-B der Fig. 7, und
- Fig. 9: eine schematische Draufsicht auf eine mittels einer zentralen Steuereinheit hydraulisch oder pneumatisch über eine zentrale Kompressionseinheit betriebenen Biogasanlage.

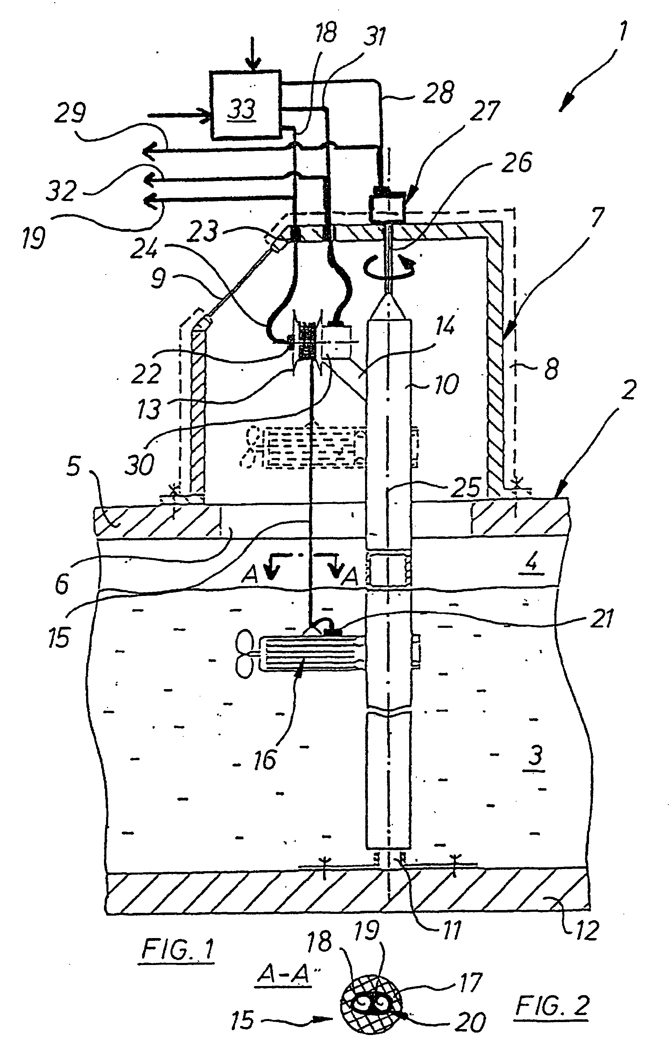

In der Fig. 1 ist ein schematischer Querschnitt durch eine Biogasanlage 1 zur Fermentation von organischen Stoffen dargestellt, die einen Fermenterbehälter 2 mit einem unteren Flüssigkeitsbereich 3 und einem darüber liegenden Gasbereich 4 aufweist.

In einer oberen Behälterwand 5 ist eine Öffnung 6 ausgebildet, um die herum ein Dom 7 dicht auf die obere Behälterwand 5 aufgesetzt und dort befestigt ist. Der Dom 7 weist an der Außenseite bevorzugt eine Wärmeisolierung 8, wie sie in der Fig. 1 strichliert dargestellt ist, sowie ein Kontrollfenster 9 auf. Der Fermenterbehälter 2 kann ganz oder wenigstens teilweise im Boden versenkt sein.

Im Fermenterbehälter 2 ist ferner ein Rührwerkträger 10 angeordnet, der mit einem unteren Trägerende an einem unteren Lager 11 an einer unteren Behälterwand 12 drehbar gelagert ist. Ein oberes Trägerende des Rührwerkträgers 10 ragt durch die Öffnung 6 hindurch in einen Innenraum des Doms 7.

Im Innenraum des Doms 7 und damit innerhalb des Fermenterbehälters 2 ist eine Seiltrommel 13 mit einer horizontalen Seiltrommelwelle über eine Konsole 14 mit dem Rührwerkträger 10 fest verbunden. Ein auf der Seiltrommel 13 aufwickelbares Seil 15 ist mit einem freien Seilende mit einem Tauchmotor-Rührgerät 16 zu dessen Höhenverstellung verbunden.

Das Tauchmotor-Rührgerät 16 umfasst einen durch ein Arbeitsmedium betreibbaren Tauchmotor. Dieser Tauchmotor kann ein Pneumatik-Motor mit Gas oder Luft als Arbeitsmedium oder alternativ auch ein Hydraulik-Motor mit einer Hydraulikflüssigkeit als Arbeitsmedium sein.

Das Seil 15 ist hier, wie dies insbesondere aus der Fig. 2 ersichtlich ist, die einen Schnitt entlang der Linie A-A der Fig. 1 zeigt, aus einem Stahlgeflecht 17 gebildet, das eine darin integrierte Zuführleitung 18 und Abführleitung 19 für das Arbeitsmedium als Bestandteile einer Energie-Leitung 20 des Tauchmotors umgibt.

Die Zuführleitung 18 ist je nach dem gewählten Arbeitsmedium an einen Hydraulik- oder Luftkompressor als Medium-Kompressionseinheit, der in der Darstellung der Fig. 1 allerdings nicht dargestellt ist, angeschlossen, und wird von dort mit dem entsprechenden Medium für eine Zuführung desselben zum Tauchmotor versorgt.

Wie dies aus der Fig. 1 weiter ersichtlich ist, ist das Seil 15 im Anbindungsbereich an das Tauchmotor-Rührgerät 16 abgehängt und lastfrei mit einer Seilschlaufe zu einem Hydraulik/Pneumatik-Anschluss 21 am Tauchmotor-Rührgerät 16 geführt.

Die Seiltrommel 13 weist eine hier lediglich äußerst schematisch dargestellte axiale Drehkupplung 22 auf, von der ausgehend ein Teil des Seils 15 in einer Schleife 24 zu einer ortsfesten und gasdichten Behälterwanddurchführung 23 verläuft und ein weiterer Teil des Seils 15 auf der Seiltrommel 13 auf- und abwickelbar ist. Durch die Schleife 24 wird eine Verschwenkung des Rührwerkträgers 10 um dessen Längsachse 25 ermöglicht, ohne dass das Seil 15 auf Zug gelangt und diese behindert.

Mit einer derartigen Seilführung wird erreicht, dass die Abführleitung 19 auf jeden Fall dicht aus dem Fermenterbehälter 2 herausgeführt ist. Bei der Verwendung von Luft als Arbeitsmedium kann die Abführleitung 19 als Abblasleitung in die Umgebung ausblasen. Bei der Verwendung eines Gases, z. B. eines Inertgases, oder einer Hydraulikflüssigkeit als Arbeitsmedium ist die Abführleitung 19 als Rückleitung vorzugsweise in einem geschlossenem Hydraulik- oder Gaskreislauf geführt und entsprechend an den zugeordneten Gas- bzw. Hydraulikkompressor angeschlossen.

Wie dies aus der Fig. 1 weiter ersichtlich ist, umfasst das obere Trägerende des Rührwerkträgers 10 endseitig als Verlängerung des Rührwerkträgers 10 eine Drehantriebswelle 26 eines ebenfalls durch ein Arbeitsmedium hydraulisch oder pneumatisch betreibbaren Drehantriebs 27. Über diesen Drehantrieb 27 ist der Rührwerkträger 10 um dessen Längsachse 25 verdrehbar in einer bestimmten Drehstellung einstellbar und festlegbar. Dazu ist die Drehantriebswelle 26 beispielsweise durch eine gasdichte Buchse als dichte Wellendurchführung durch eine obere Domwand geführt. Der Drehantrieb 27 wird hier durch einen pneumatisch oder hydraulisch betreibbarer Schwenkmotor gebildet, der entsprechend eine Zuführleitung 28 sowie eine Abführleitung 29 für das Arbeitsmedium umfasst.

Die Seillänge des Seils 15 ist insgesamt so gewählt, dass das Tauchmotor-Rührgerät 16 maximal bis knapp vor die untere Behälterwand 12 abgesenkt werden kann. Zur Höhenverstellung der Seiltrommel 13 ist hier ein separater hydraulisch oder pneumatisch betreibbarer Seiltrommelantrieb 30 vorgesehen, der wiederum eine Zuführleitung 31 und eine Abführleitung 32 für das Arbeitsmedium aufweist.

Sowohl die Zuführ- und Abführleitung 28, 29 des Drehantriebs 27 als auch die Zuführ- und Abführleitung 31, 32 des Seiltrommelantriebs 30 sind dabei entsprechend dem Seil 15 zumindest im Innenraum des Doms 7 und damit dem Fermenterbehälter 2 von einem Stahlgeflechtmantel ummantelt und werden erst außerhalb des Doms 7 und des Fermenterbehälters 2 als separate Leitungen weitergeführt.

Wie dies in der Fig. 1 lediglich schematisch dargestellt ist, können sämtliche Antriebseinheiten über eine zentrale Steuereinheit 33, die einer elektrisch betreibbaren, zentralen Kompressionseinheit zugeordnet sein kann, betrieben werden.

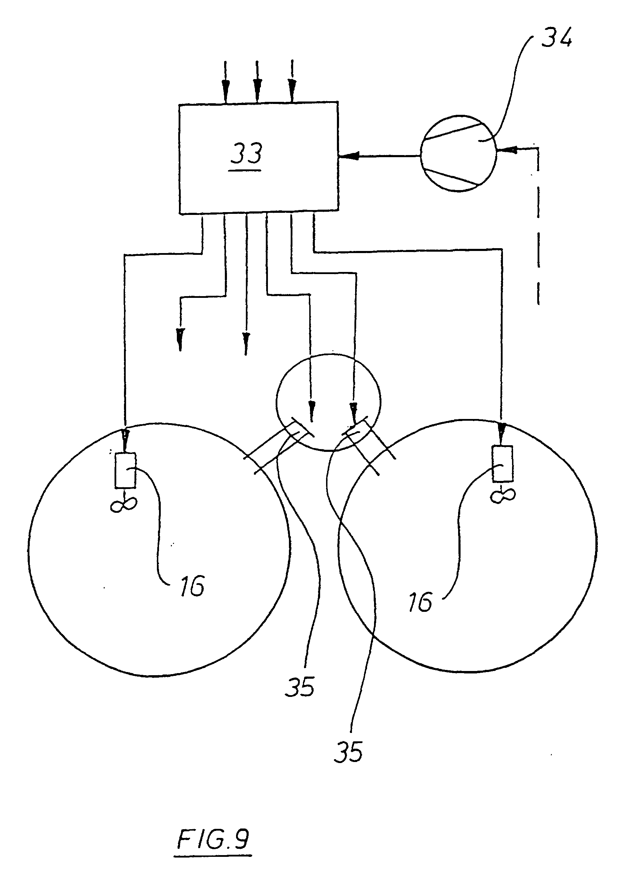

In der Fig. 9 ist eine schematische Draufsicht auf die hier lediglich beispielhaft mit zwei Fermenterbehälter 2 betriebene Biogasanlage 1 gezeigt. Über die Steuereinheit 33 werden hier alle Hydraulik- oder Pneumatikmotoren von einer zentralen, elektrisch betreibbaren Kompressionseinheit 34 versorgt. Die elektrisch betriebene Kompressionseinheit 34 ist hier vorteilhaft weit außerhalb entfernt vom Fermenterbehälter 2 angeordnet, so dass im Fermenterbehälter 2 der Explosionsschutz durch die Verwendung von Hydraulik- und/oder Pneumatikleitungen für die entsprechend zugeordneten Hydraulik- und/oder Pneumatikmotoren einfach beherrschbar ist. Die weiteren Hydraulik- und/oder Pneumatikmotoren können z. B. für Pumpen und/oder Bodenräumgeräte und/oder Schutzvorrichtungen und/oder Schieber an Rohrleitungen vorgesehen sein. Mit Bezugszeichen 35 ist in Fig. 9 hier beispielhaft der Ort gekennzeichnet, in dem Schieber samt Motoren an Rohrleitungen angeordnet sein können.

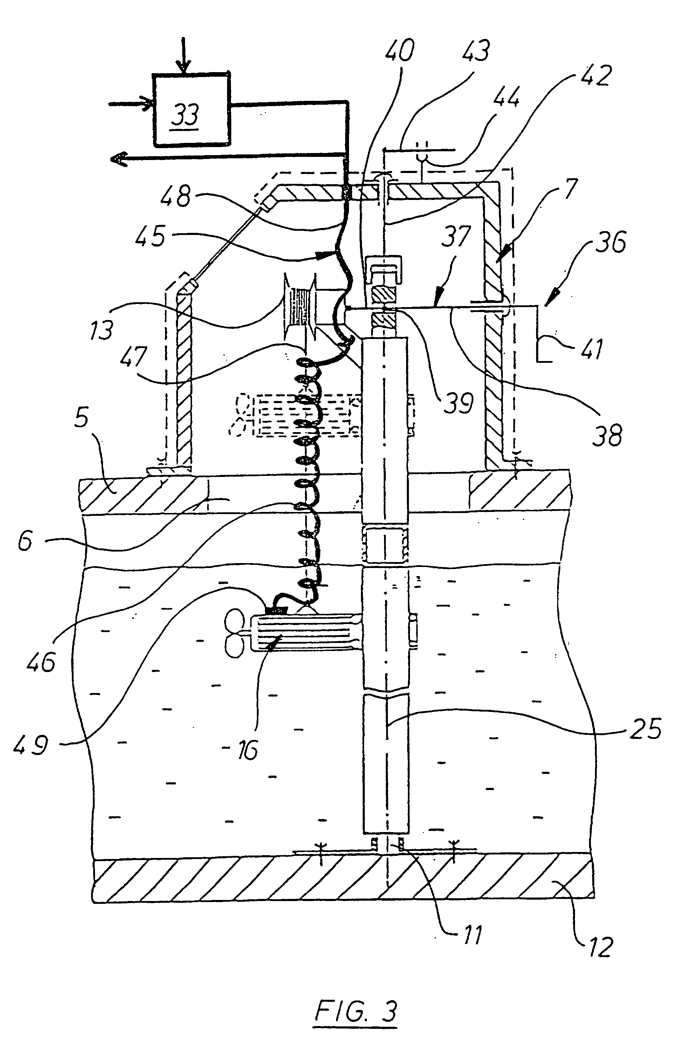

In der Fig. 3 ist ein alternativer Aufbau dargestellt, wobei in den Fig. 1 und 3 gleiche Teile mit jeweils gleichen Bezugszeichen bezeichnet und nicht mehr näher erläutert werden.

In der Fig. 3 ist anstelle des hydraulisch oder pneumatisch betreibbaren Seiltrommelantrieb 30 gemäß Fig. 1 ein manuell bedienbarer Seiltrommelantrieb 36 mit einer Gelenkwelle 37 vorgesehen. Dazu ist eine ortsfeste Welle 38 der Gelenkwelle 37 des Seiltrommelantriebs 36 durch eine gasdichte Buchse als dichte Wellendurchführung durch eine seitliche Domwand geführt. Ein Gelenk 39 der Gelenkwelle 37 dient als Mittel zur Umlenkung der Bewegungsübertragungsrichtung, wobei die Umlenkung in der Rührwerkträger-Längsachse 25 erfolgt. Das Gelenk 39 ist z. B. ein Einfach-Wellengelenk, das in einem Gelenklager am oberen Trägerende geführt und gelagert ist, wobei sich an das Gelenk 39 gegenüberliegend zur ortsfesten Welle 38 eine Seiltrommelwelle 40 der Gelenkwelle 37 anschließt. Diese Seiltrommelwelle 40 ist mit der Seiltrommel 13 drehübertragend verbunden. Zur Betätigung der Gelenkwelle 37 ist eine Kurbel 41 vorgesehen, die mit der ortsfesten Welle 38 verbunden ist. Zur Höhenverstellung kann somit hier über die Kurbel 41 eine Drehbewegung über die Gelenkwelle 37 auf die Seiltrommel 13 übertragen werden. Je nach der Drehrichtung kann das Tauchmotor-Rührgerät 16 gehoben bzw. gesenkt werden. Im Falle einer Verschwenkung des Rührwerkträgers 10 um dessen Längsachse 25 kann die Seiltrommelwelle 40 in einer Ebene senkrecht zur vertikalen Längsachse 25 relativ zu der ortsfesten Welle 38 verschwenkt werden.

Ferner ist in der Fig. 3 anstelle des Drehantriebs 27 der Fig. 1 zur Verschwenkung des Rührwerkträgers 10 eine Drehverstellung von Hand möglich. Dazu ist eine ortsfeste Drehantriebswelle 42 mit einem oberen Trägerende des Rührwerkträgers 10 verbunden, wobei die Drehantriebswelle 42 durch eine gasdichte Buchse als dichte Wellenführung durch eine obere Domwand geführt ist. Zum manuellen Verschwenken des Rührwerkträgers 10 ist ein gelenkig mit der Drehantriebswelle 42 verbundener, aufschwenkbarer Stellhebel 43 vorgesehen. Dieser Stellhebel 43 ist über eine Gabelraste 44 an der oberen Domwand in einer bestimmten Drehstellung des Rührwerkträgers 10 festlegbar.

Ein auf die Seiltrommel 13 aufwickelbares Seil 47 ist mit einem freien Seilende mit dem Tauchmotor-Rührgerät 16 zu dessen Höhenverstellung verbunden. Auch hier ist die Seilwelle so gewählt, dass das Tauchmotor-Rührgerät 16 maximal bis knapp vor die untere Behälterwand 12 abgesenkt werden kann.

Der Tauchmotor des Tauchmotor-Rührgeräts 16 ist auch hier wiederum als ein durch ein Arbeitsmedium betreibbarer Hydraulik- oder Pneumatik-Motor ausgebildet, dessen Energie-Leitung gasdicht durch eine obere Domwand geführt ist und in einer mehr oder weniger großen Schleife 48 bis zu der Konsole 14 geführt ist, wo die Energie-Leitung 45 festgelegt ist. Durch diese Schleife 48 ist bei einer Verschwenkung des Rührwerkträgers 10 sichergestellt, dass die Verschwenkung nicht durch die Energie-Leitung 45 behindert wird. Von der Konsole 14 ausgehend ist die Energie-Leitung in losen Schlaufen 46 um das Seil 47 gewickelt und bis zu einem Hydraulik/Pneumatik-Anschluss 49 am Tauchmotor-Rührgerät 16 geführt, wobei die Schlaufen 46 je nach der Verstellposition des Tauchmotor-Rührgeräts 16 mehr oder weniger zusammengeschoben sind.

Die Energie-Leitung 45 umfasst auch hier wiederum eine in der Fig. 3 nicht näher dargestellte Zuführleitung sowie eine Abführleitung für das Arbeitsmedium. Diese zwei Leitungen können beispielsweise entsprechend der in der Fig. 2 dargestellten Ausführungsform in einem Stahlgeflechtmantel integriert sein oder aber auch als zwei separate Leitungen ausgebildet sein, die gegebenenfalls miteinander durch z. B. Verkleben oder durch Leitungsschellen miteinander verbunden sind. Die Festlegung der Energie-Leitung 45 an der Konsole 14 erfolgt dabei z. B. ebenfalls mittels einer Schelle.

Auch hier ist somit entsprechend dem Aufbau der Fig. 1 ein vorteilhafter Explosionsschutz realisierbar.

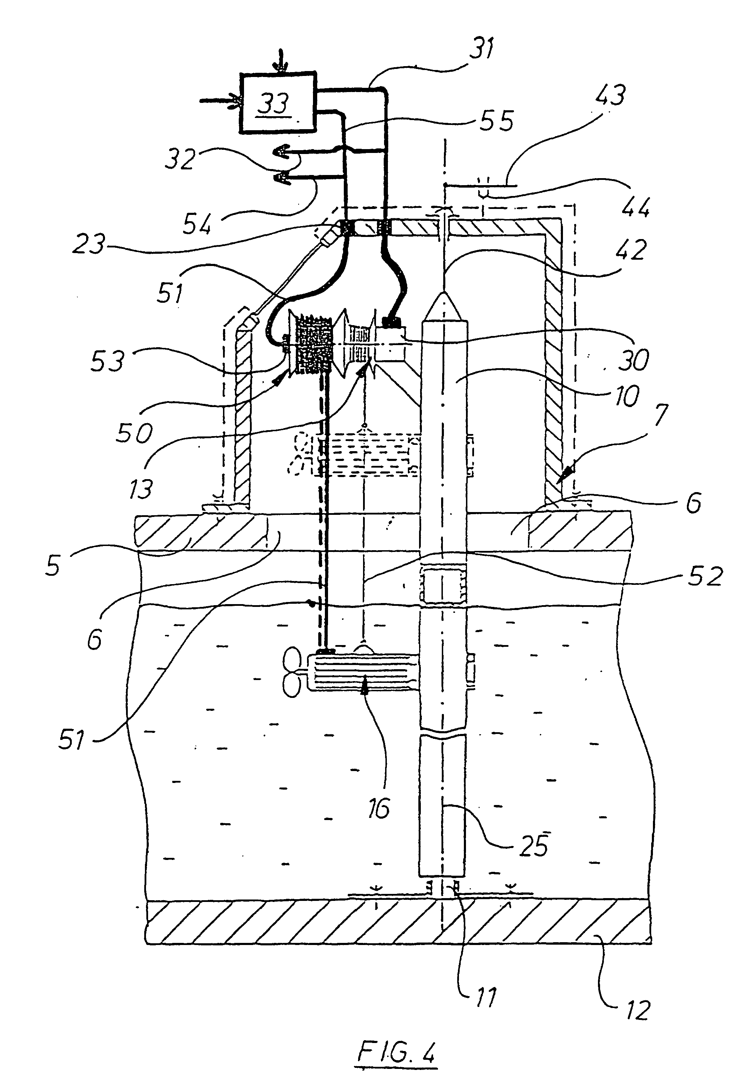

In der Fig. 4 ist eine weitere alternative Ausführungsform, die ebenfalls die in Verbindung mit den Fig. 1 und 3 geschilderten Vorteile aufweist, dargestellt, bei der der Rührwerkträger 10 entsprechend der Fig. 3 wieder mittels des Stellhebels 43 von Hand verschwenkbar ist. Die Seiltrommel 13 ist hier wieder entsprechend dem Ausführungsbeispiel der Fig. 1 hydraulisch oder pneumatisch mittels des Seiltrommelantriebs 30, das heißt mittels eines Hydraulikoder eines Pneumatikmotors, antreibbar. Mit dieser Seiltrommel 13 ist eine Energie-Leitungs-Trommel 50 gleichachsig und drehbewegungsübertragend gekoppelt, auf der entsprechend und zugeordnet dem Seilwicklungsstand eine Energie-Leitung 51 aufwickelbar ist. Die Einstellung des Wickelgrades der Energie-Leitung 51 wird dabei so gewählt, dass in jeder Verstellposition des Tauchmotor-Rührgeräts 16 nur ein Zug auf ein der Seiltrommel 13 zugeordnetes Seil 52 wirkt, die Energie-Leitung 51 jedoch ohne jeden größeren mechanischen Zug entsprechend ab- und aufgewickelt werden kann.

Die Energie-Leitung 51 weist eine hier lediglich äußerst schematisch dargestellte axiale Drehkopplung 53 mit einem nicht drehenden Kupplungsteil auf, an den der zur ortsfesten Behälterwanddurchführung 23 geführte Teil der Energie-Leitung 51 angeschlossen ist, während am anderen, drehbaren Kupplungsteil der auf der Energie-Leitungs-Trommel 50 auf- und abwickelbare Teil der Energie-Leitung 51 angeschlossen ist.

Durch die gleichachsige und drehbewegungsübertragende Drehkopplung der Seiltrommel 13 mit der Energie-Leitungs-Trommel 50 wird erreicht, dass zur Verdrehung der selben nur ein einziger Antrieb, nämlich der Seiltrommelantrieb 30 benötigt wird.

Die Energie-Leitung 51 weist auch hier wiederum eine Abführleitung 54 sowie eine Zuführleitung 55 auf, mit der der Tauchmotor des Tauchmotor-Rührgeräts 16 hydraulisch oder pneumatisch mittels eines geeigneten Arbeitsmediums betrieben werden kann.

Auch hier können die Zuführleitung 55 und die Abführleitung 54 wieder wenigstens bereichsweise in einem einzigen Seil mit z. B. Stahlgeflechtummantelung integriert sein, beispielsweise gemäß in der Fig. 2 dargestellten Art und Weise, oder aber auch zwei separate Leitungen vorgesehen sein, die gegebenenfalls wenigstens in Teilbereichen über z. B. Leitungsschellen miteinander verklippst und/oder miteinander verklebt werden können.

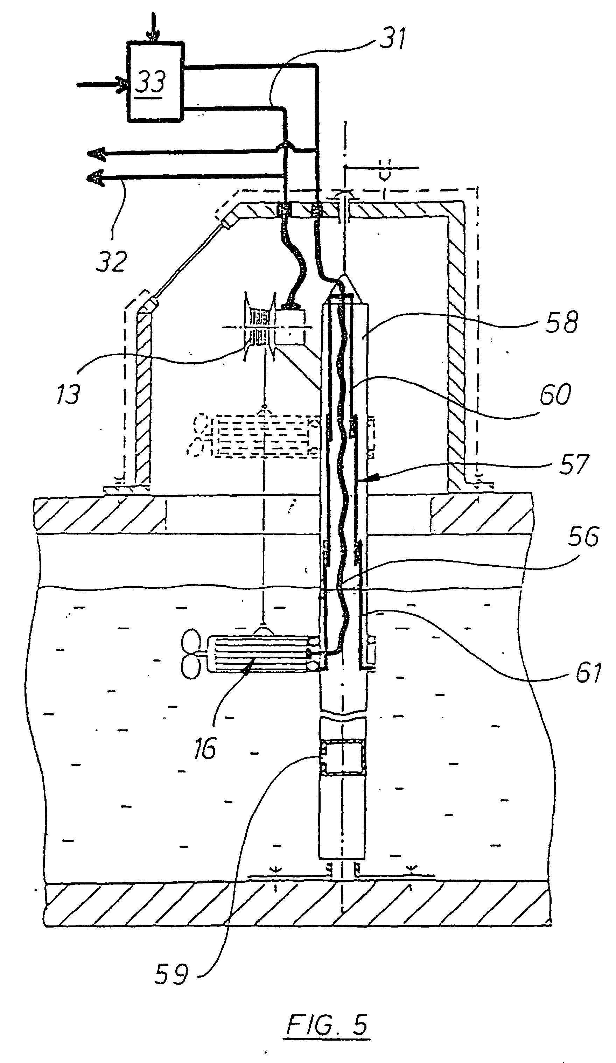

In der Fig. 5 ist eine weitere alternative Ausführungsform gezeigt, bei der die Seiltrommel 13 hydraulisch oder pneumatisch in der in Verbindung mit der Fig. 4 und der Fig. 1 bereits näher erläuterten Art und Weise angetrieben wird, so dass hierauf nicht mehr eingegangen wird. Im Unterschied zu den vorangehenden Ausführungsformen ist hier eine Energie-Leitung 56 für einen hydraulisch oder pneumatisch durch ein Arbeitsmedium betreibbaren Tauchmotor des Tauchmotor-Rührgeräts 16 in einem aus mehreren Teleskoprohren bestehenden Teleskoprohr 57 geführt. Das Teleskoprohr 57 ist hier in einem Rührwerkträger 58 angebracht, der, wie dies aus der Fig. 5 ersichtlich ist, einen Längsschlitz 59 aufweist, durch den die Verbindung zum Motor-Rührgerät 16 hergestellt ist. Das Teleskoprohr 57 ist hier ferner nach unten hin offen ausgebildet.

Das Teleskoprohr 57 erstreckt sich hier vertikal im Inneren des Rührwerkträgers 58, wobei das obere Teleskoprohr 60 in einem oberen Bereich festgelegt ist und das untere Teleskoprohr 61 unmittelbar oder mittelbar mit dem Tauchmotor-Rührgerät 16 verbunden ist. Dadurch wird erreicht, dass das Teleskoprohr 57 in einer unteren Position des Tauchmotor-Rührgeräts 16 vollständig ausgezogen ist, wie dies in der Fig. 5 lediglich beispielhaft und schematisch dargestellt ist, während das Teleskoprohr 57 in einer oberen Position des Tauchmotor-Rührgeräts 16 zusammengeschoben ist.

Die Funktionsweise des hydraulisch oder pneumatisch betriebenen Tauchmotors des Tauchmotor-Rührgeräts 16 entspricht derjenigen, wie sie bereits in Verbindung mit den Fig. 1 bis 4 näher erläutert worden ist, so dass hierauf ebenfalls nicht mehr näher darauf eingegangen wird.

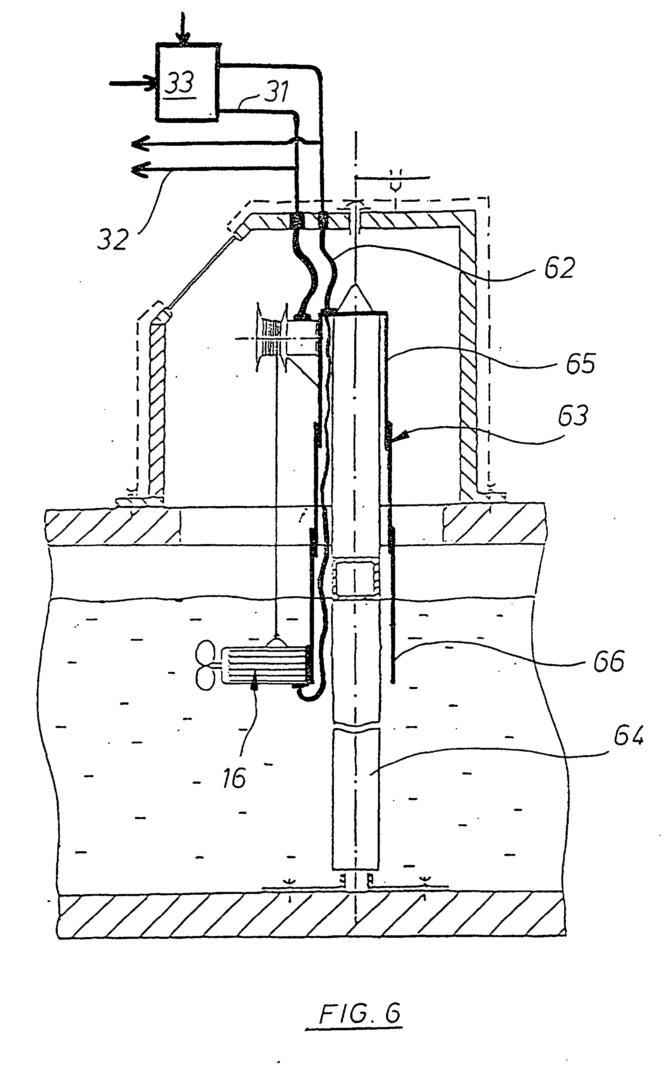

In Fig. 6 ist eine zu Fig. 5 alternative Ausführungsform dargestellt, bei der eine Energie-Leitung 62 ebenfalls in einem aus mehreren Teleskoprohren bestehenden Teleskoprohr 63 geführt ist. Im Unterschied zur Ausführungsform nach Fig. 5 umgibt hier jedoch das Teleskoprohr 63 einen Rührwerkträger 64, wobei die Energie-Leitung 62 hier zwischen dem Rührwerkträger 64 und einer Teleskoprohrinnenwand geführt ist.

Auch im Ausführungsbeispiel nach Fig. 6 ist ein oberes Teleskoprohr 65 in einem oberen Bereich des Rührwerkträgers 64 festgelegt, während ein unteres Teleskoprohr 66 unmittelbar oder mittelbar mit dem Tauchmotor-Rührgerät 16 verbunden ist, so dass das Teleskoprohr 63 in einer unteren Position des Tauchmotor-Rührgeräts 16 ausgezogen und in einer oberen Position zusammengeschoben ist.

Sowohl im Ausführungsbeispiel nach Fig. 5 als auch im Ausführungsbeispiel nach Fig. 6 kann die Energie-Leitung 51 bzw. 56 als Seil ausgebildet sein, das entsprechend der Ausführungsform gemäß Fig. 2 mit einem Stahlmantel ummantelt ist. Alternativ dazu können aber auch separate oder miteinander verklippste bzw. verklebte Leitungen vorgesehen sein.

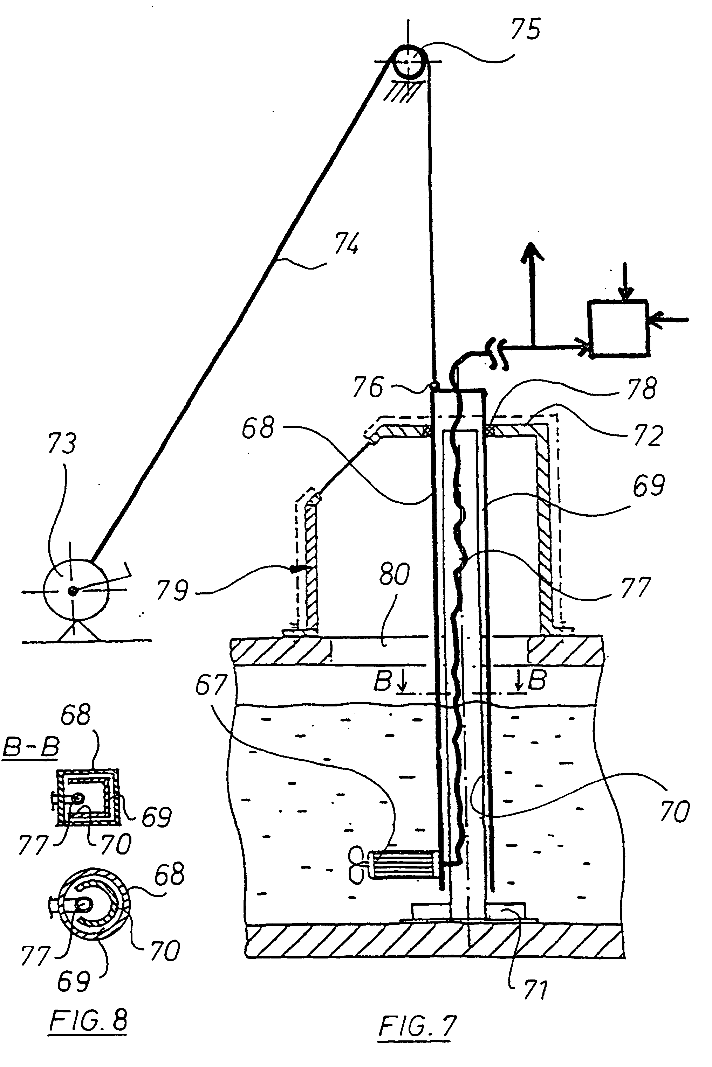

In der Fig. 7 ist eine weitere alternative Ausführungsform dargestellt, bei der das Tauchmotor-Rührgerät 67 an einem endseitigen Bereich eines Stülprohrs 68 angeordnet ist. Dieses Stülprohr 68 ist in der Darstellung der Fig. 7 teleskopartig mit einem Spaltabstand 69 über einen vertikal im Fermenterbehälter angebrachten, stützenförmigen Rührwerkträger 70 gestülpt und daran in Rührwerkträger-Längsrichtung zur Einstellung unterschiedlicher Tauchmotor-Höhenpositionen im Fermenterbehälter verschiebbar geführt. In der in der Fig. 7 gezeigten Darstellung befindet sich das Stülprohr in einer nahezu vollständig abgesenkten Stellung, bei der das Tauchmotor-Rührgerät 67 nahe dem Bodenbereich des Fermenterbehälters angeordnet ist. Um den Rührwerkträger 70 stabil abzustützen, ist am Boden des Fermenterbehälters eine stabile Lagerplatte 71 vorgesehen. Um das Tauchmotor-Rührgerät 67 in einer Horizontalebene in gewissen Winkelbereichen verstellen zu können, ist bei einem in der Fig. 8 dargestellten kreisförmigen Querschnittsprofil des Stülprohrs und einem C-förmigen Querschnittsprofil des Rührwerkträgers lediglich ein Verdrehen des Stülprohrs 68 erforderlich, wobei der Schwenkwinkel durch den offenen Profilquerschnitt des Rührwerkträgers begrenzt und definiert ist. Bei einem in der Fig. 8 alternativ dargestellten in etwa rechteckförmigen Querschnittsprofil des Stülprohrs 68 und des Rührwerkträgers 70, die nicht gegeneinander verdrehbar sind, ist es dagegen erforderlich, dass im Bereich der Lagerplatte 71 ein Schwenklager zur Verschwenkung des Tauchmotor-Rührgeräts 67 in einer Horizontalebene vorgesehen ist.

Wie dies aus der Fig. 7 weiter ersichtlich ist, ist das Stülprohr 68 so dimensioniert, dass es in jeder Tauchmotor-Höhenposition mit wenigstens einem Teilbereich dicht durch eine Dom-Wand 72 des Fermenterbehälters geführt ist. Das Stülprohr 68 und damit das Tauchmotor-Rührgerät 67 sind mittels einer außerhalb des Fermenterbehälters angeordneten Seilwinde 73 anhebbar oder absenkbar. Dazu ist ein Seil 74 der Seilwinde über eine in etwa auf maximaler Stülprohr-Hubhöhe angeordnete, ortsfeste Umlenkrolle 75 zu einer am oberen Stülprohrende angeordneten Seilanbindungsstelle 76 geführt.

Wie dies aus der Fig. 7 in Verbindung mit der Fig. 8 weiter ersichtlich ist, ist eine Energieleitung 77, die bereits zuvor in Verbindung mit den anderen Ausführungsbeispielen näher erläutert worden ist, vom Tauchmotor-Rührgerät 67 her kommend dicht durch das Stülprohr 68 hindurchgeführt und verläuft innerhalb des offenen Profilquerschnitts nach oben, wo es aus dem Rührwerk-Träger 70 und dem Stülprohr 68 in der zuvor beschriebenen Art und Weise herausgeführt ist.

Das Stülprohr 68 ist mittels einer herkömmlichen Stopfbuchse 78 gasdicht durch die Dom-Wand 72 hindurchgeführt sowie ggf. mit Fett abgeschmiert. Eine Abdichtung vom Inneren des Stülprohrs 68 bzw. Rührwerkträgers 70 her ist hier nicht erforderlich, da sich in diesen beiden Rohren ein Flüssigkeitsspiegel als Siffondichtung entsprechend dem Flüssigkeitsspiegel im Fermenterbehälter einstellt.

Bei einer Betätigung der Seilwinde 73 wird über das Seil 74 eine Zugkraft auf das Stülprohr 68 aufgebracht, so dass das Tauchmotor-Rührgerät 67 je nach Bedarf in eine gegenüber der Darstellung der Fig. 7 höhere Tauchmotor-Rührgerät-Position, so zum Beispiel in den Bereich des Doms 79 für Wartungsarbeiten anhebbar ist, bei denen eine Rührwerköffnung 80 mittels zum Beispiel Brettern nach unten hin abdichtbar und dann der Dom 79 betretbar ist.

## Patentansprüche

1. Biogasanlage zur Fermentation von organischen Stoffen
mit mindestens einem Fermenterbehälter mit einem unteren Flüssigkeitsbereich und einem darüberliegenden Gasbereich,
mit mindestens einem im Fermenterbehälter angeordneten Tauchmotor-Rührgerät,
mit einer Energie-Leitung, die dicht durch eine Behälterwand und weiter durch den Gasbereich zum Tauchmotor des Tauchmotor-Rührgeräts geführt ist,
**dadurch gekennzeichnet,**
**dass** der Tauchmotor ein durch ein Arbeits-Medium betreibbarer Motor ist,
**dass** die Energie-Leitung (20; 45; 51; 56; 62; 77) einerseits eine Rohroder Schlauchleitung als Zuführleitung (18; 55) für das Medium umfasst, die an eine Medium-Kompressionseinheit (34) angeschlossen ist, und andererseits eine Rohr- oder Schlauchleitung als Abführleitung (19; 54) für das Medium umfasst,
**dass** die Abführleitung (19; 54) vom Tauchmotor durch den Gasbereich (4) und dicht durch eine Behälterwand aus dem Fermenterbehälter (2) herausgeführt ist, und
**dass** die Medium-Kompressionseinheit (34) elektrisch betreibbar ist und außerhalb des Fermenterbehälters (2) angeordnet ist.

2. Biogasanlage nach Anspruch 1, **dadurch gekennzeichnet,**
**dass** der Tauchmotor ein Hydraulik-Motor ist, wobei das Medium eine Hydraulikflüssigkeit ist und die Kompressionseinheit ein Hydraulik-Kompressor (34) ist, und
**dass** die Abführleitung (19; 54) als Rückleitung in einem geschlossenen Hydraulikkreislauf an den Hydraulik-Kompressor (34) angeschlossen ist.

3. Biogasanlage nach Anspruch 1, **dadurch gekennzeichnet,**
**dass** der Tauchmotor ein Pneumatik-Motor ist, wobei das Medium Luft ist und die Kompressionseinheit (34) ein Luftkompressor ist, und
**dass** die Abführleitung (19; 54) als Abblasleitung dicht aus dem Fermenterbehälter (2) geführt ist.

4. Biogasanlage nach einem der Ansprüche 1 bis 3, **dadurch gekennzeichnet,**
**dass** weitere Hydraulik-Motoren und/oder Pneumatik-Motoren als Pumpenmotoren und/oder Bodenräumgeräte-Motoren und/oder Schutzvorrichtungsmotoren und/oder Schiebermotoren an Rohrleitungen und/oder Hubmotoren (30) und/oder Schwenkmotoren (27) verwendet sind, und
**dass** alle Motoren von einer zentralen Kompressionseinheit (34) über eine Steuereinheit (33) betreibbar sind.

5. Biogasanlage nach einem der Ansprüche 1 bis 4, **dadurch gekennzeichnet,**
**dass** das Tauchmotor-Rührgerät (16) an einer Hubvorrichtung vertikal verstellbar angeordnet ist,
**dass** die Hubvorrichtung einen vertikal im Behälter angebrachten, stützenförmigen Rührwerkträger (10; 58; 64) umfasst, an dem das Tauchmotor-Rührgerät (16) verschiebbar gehalten ist, und
**dass** im oberen Bereich des Rührwerkträgers (10; 58; 64) als Bestandteil einer betätigbaren Seilwinde eine Seiltrommel (13) mit einer Seilverbindung durch ein aufwickelbares Seil (15; 47; 52) zum Tauchmotor-Rührgerät (16) angeordnet ist.

6. Biogasanlage nach Anspruch 5, **dadurch gekennzeichnet,**
**dass** die Energie-Leitung (45) im oberen Bereich des Rührwerkträgers (10) festgelegt ist, und
**dass** die Energie-Leitung (45) in losen Schlaufen (46) um das Seil (47) gewickelt ist, wobei die Schlaufen (46) je nach der Verstellposition des Tauchmotor-Rührgeräts (16) mehr oder weniger zusammengeschoben sind.

7. Biogasanlage nach Anspruch 5, **dadurch gekennzeichnet,**
**dass** für die Energie-Leitung (51) eine betätigbare Trommel als Energie-Leitungs-Trommel (50) vorgesehen ist, auf der entsprechend und zugeordnet dem Seilwicklungsstand die Energie-Leitung (51) aufwickelbar ist, und
**dass** die Energie-Leitungs-Trommel (50) eine axiale Drehkupplung (53) aufweist, von der einerseits ein Teil der Energie-Leitung (51) zur ortsfesten Behälterwanddurchführung verläuft und andererseits ein Teil der Energie-Leitung (51) auf der Energie-Leitungs-Trommel (50) aufwickelbar und abwickelbar ist.

8. Biogasanlage nach Anspruch 7, **dadurch gekennzeichnet, dass** die Seiltrommel (13) und die Energie-Leitungs-Trommel (50) gleichachsig und drehbewegungsübertragend gekoppelt sind.

9. Biogasanlage nach Anspruch 7, **dadurch gekennzeichnet,**
**dass** die Seiltrommel (13) zugleich die Energie-Leitungs-Trommel ist, und
**dass** die Energie-Leitung (20) und das Seil (15) über ihre Längen verbunden sind.

10. Biogasanlage nach Anspruch 9, **dadurch gekennzeichnet, dass** das Seil (15) aus einem Stahlgeflecht (17) gebildet ist, das die darin integrierte Zuführleitung (18) und/oder die Abführleitung (19) der Energie-Leitung (20) umgibt.

11. Biogasanlage nach einem der Ansprüche 5 bis 10, **dadurch gekennzeichnet,**
**dass** das Seil und/oder die Energie-Leitung (56; 62) in einem aus mehreren Teleskoprohren bestehenden Teleskoprohr (57; 63) geführt sind, und
**dass** sich das Teleskoprohr (57; 63) vertikal entlang des Rührwerkträgers (58; 64) erstreckt, wobei das obere Teleskoprohr (60; 65) in einem oberen Bereich des Fermenterbehälters (2) festgelegt ist und das untere Teleskoprohr (61; 66) unmittelbar oder mittelbar mit dem Tauchmotor-Rührgerät (16) verbunden ist, so dass das Teleskoprohr (57; 63) in einer unteren Position des Tauchmotor-Rührgeräts (16) ausgezogen und in einer oberen Position zusammengeschoben ist.

12. Biogasanlage nach Anspruch 11, **dadurch gekennzeichnet, dass** das Teleskoprohr (57) im Rührwerkträger (58) angebracht ist und der Rührwerkträger (58) einen Längsschlitz (59) aufweist durch den die Verbindung zum Tauchmotor-Rührgerät (16) hergestellt ist.

13. Biogasanlage nach Anspruch 11, **dadurch gekennzeichnet, dass** das Teleskoprohr (63) den Rührwerkträger (64) umgibt und das Seil und/oder die Energie-Leitung (62) zwischen dem Rührwerkträger (64) und der Teleskoprohrinnenwand geführt sind.

14. Biogasanlage nach einem der Ansprüche 1 bis 4, **dadurch gekennzeichnet,**
**dass** das Tauchmotor-Rührgerät (67) an einem Stülprohr (68), vorzugswiese an einem unteren Stülprohrende, angeordnet ist, das teleskopartig, vorzugsweise mit einem Spaltabstand (69), über einen vertikal im Behälter angebrachten, stützenförmigen Rührwerkträger (70) stülpbar und daran in Rührwerkträger-Längsrichtung zur Einstellung unterschiedlicher Tauchmotor-Höhenpositionen im Fermenterbehälter verschiebbar geführt ist,
**dass** das Stülprohr (68) in jeder Tauchmotor-Höhenposition mit wenigstens einem Teilbereich des Stülprohrs (68) dicht durch den Fermenterbehälter geführt ist, und
**dass** die Energie-Leitung (77) im Stülprohr (68) und/oder im Rührwerkträger (70) geführt ist.

15. Biogasanlage nach Anspruch 14, **dadurch gekennzeichnet, dass** das Stülprohr (68) und damit das Tauchmotor-Rührgerät (67) mit einer außerhalb des Fermenterbehälters anordenbaren Hubvorrichtung (73) anhebbar oder absenkbar ist, vorzugsweise mit einer ortsfesten Seilwinde, deren Seil (74) über einen in etwa wenigstens auf maximaler Stülprohr-Hubhöhe angeordneten, ortsfesten Umlenkpunkt, vorzugsweise eine Umlenkrolle (75), zu einer am oberen Stülprohrende angeordneten Seilanbindungsstelle (76) geführt ist.

16. Biogasanlage nach Anspruch 14 oder Anspruch 15, **dadurch gekennzeichnet, dass** das Stülprohr (68) einen geschlossenen Profilquerschnitt und der Rührwerkträger einen zum Tauchmotor hin ausgerichteten offenen Profilquerschnitt aufweist.

17. Biogasanlage nach einem der Ansprüche 1 bis 16, **dadurch gekennzeichnet,**
**dass** oberhalb des Tauchmotor-Rührgeräts (16) in der Fermenterbehälter-Deckenwand (5) eine Rührwerköffnung (6; 80) enthalten ist,
**dass** über der Rührwerköffnung (6) gasdicht ein Service-Dom (7) angebracht ist, in dessen Bereich der Rührwerkträger (10; 58; 64) mit seinem oberen Ende ragt und in den das Tauchmotor-Rührgerät (16; 67) hochziehbar ist, und
**dass** wenigstens eine Energie-Leitung (20; 45; 51; 56; 62; 77) gasdicht durch eine Service-Dom-Wand in den Fermenterbehälter (2) eingeführt ist.

## Claims

1. Biogas plant for the fermentation of organic materials, having at least one fermentation vessel with a lower liquid region and a gas region situated above it, having at least one submersible motorized stirrer arranged in the fermentation vessel, having a power line which is guided in a sealed manner through a vessel wall and on through the gas region to the submersible motor of the submersible motorized stirrer, **characterized in that** the submersible motor is a motor which can be operated by a working medium, **in that** the power line (20; 45; 51; 56; 62; 77), on the one hand, comprises a pipeline or hose line as the feed line (18; 55) for the medium, which line is connected to a medium-compression unit (34), and, on the other hand, comprises a pipeline or hose line as a removal line (19; 54) for the medium, **in that** the removal line (19; 54) is guided from the submersible motor through the gas region (4) and in a sealed manner through a vessel wall out of the fermentation vessel (2), and **in that** the medium-compression unit (34) can be operated electrically and is arranged outside the fermentation vessel (2).

2. Biogas plant according to Claim 1, **characterized in that** the submersible motor is a hydraulic motor, the medium being a hydraulic fluid and the compression unit being a hydraulic compressor (34), and **in that** the removal line (19; 54) is connected as a return line in a closed hydraulic circuit to the hydraulic compressor (34).

3. Biogas plant according to Claim 1, **characterized in that** the submersible motor is a pneumatic motor, the medium being air and the compression unit (34) being an air compressor, and **in that** the removal line (19; 54) is guided as a blow-off line in a sealed manner out of the fermentation vessel (2).

4. Biogas plant according to one of Claims 1 to 3, **characterized in that** further hydraulic motors and/or pneumatic motors are used as pump motors and/or bottom-sweeping motors and/or protection-device motors and/or slide-valve motors on pipelines and/or hoisting motors (30) and/or pivoting motors (27), and **in that** all of the motors can be operated by a central compression unit (34) via a control unit (33).

5. Biogas plant according to one of Claims 1 to 4, **characterized in that** the submersible motorized stirrer (16) is arranged in a vertically adjustable manner on the hoisting apparatus, **in that** the hoisting apparatus comprises a pillar-shaped stirring-mechanism support (10; 58; 64) which is fitted vertically in the vessel and on which the submersible motorized stirrer (16) is held in a displaceable manner, and **in that** a cable drum (13) having a cable connection to the submersible motorized stirrer (16) via a coilable cable (15; 47; 52) is arranged in the upper region of the stirring-mechanism support (10; 58; 64) as part of an actuable cable winch.

6. Biogas plant according to Claim 5, **characterized in that** the power line (45) is fixed in the upper region of the stirring-mechanism support (10), and **in that** the power line (45) is coiled in loose loops (46) around the cable (47), the loops (46) being pushed together to a greater or lesser extent depending in each case on the adjustment position of the submersible motorized stirrer (16).

7. Biogas plant according to Claim 5, **characterized in that** an actuable drum is provided as the power-line drum (50) for the power line (51) and the power line (51) can be coiled on it in a manner which is appropriate and related to the cable-coiling state, and **in that** the power-line drum (50) has an axial rotational coupling (53) from which, on one side, part of the power line (51) runs through the positionally fixed vessel-wall bushing and, on the other side, part of the power line (51) can be coiled onto and uncoiled from the power-line drum (50).

8. Biogas plant according to Claim 7, **characterized in that** the cable drum (13) and the power-line drum (50) are coupled coaxially and in a manner such that they transmit rotational movement.

9. Biogas plant according to Claim 7, **characterized in that** the cable drum (13) is at the same time the power-line drum, and **in that** the power line (20) and the cable (15) are connected over their lengths.

10. Biogas plant according to Claim 9, **characterized in that** the cable (15) is formed from a steel mesh (17) which surrounds the feed line (18) and/or the removal line (19) of the power line (20), which lines are integrated in the said steel mesh.

11. Biogas plant according to one of Claims 5 to 10, **characterized in that** the cable and/or the power line (56; 62) are guided in a telescopic pipe (57; 63), which comprises a plurality of telescopic pipes, and **in that** the telescopic pipe (57; 63) extends vertically along the stirring-mechanism support (58; 64), the upper telescopic pipe (60; 65) being fixed in an upper region of the fermentation vessel (2) and the lower telescopic pipe (61; 66) being connected directly or indirectly to the submersible motorized stirrer (16), with the result that the telescopic pipe (57; 63) is extended in a lower position of the submersible motorized stirrer (16) and is pushed together in an upper position.

12. Biogas plant according to Claim 11, **characterized in that** the telescopic pipe (57) is fitted in the stirring-mechanism support (58), and the stirring-mechanism support (58) has a longitudinal slot (59) through which the connection to the submersible motorized stirrer (16) is produced.

13. Biogas plant according to Claim 11, **characterized in that** the telescopic pipe (63) surrounds the stirring-mechanism support (64) and the cable and/or the power line (62) are guided between the stirring-mechanism support (64) and the inner wall of the telescopic pipe.

14. Biogas plant according to one of Claims 1 to 4, **characterized in that** the submersible motorized stirrer (67) is arranged on an inverted pipe (68), preferably at a lower end of the inverted pipe, which can be inverted telescopically, preferably with a clearance (69), over a pillar-shaped stirring-mechanism support (70), which is fitted vertically in the vessel, and is guided displaceably thereon in the longitudinal direction of the stirring-mechanism support in order to set different submersible-motor height positions in the fermentation vessel, **in that**, in every submersible-motor height position, the inverted pipe (68) is guided with at least a subregion of the inverted pipe (68) in a sealed manner through the fermentation vessel, and **in that** the power line (77) is guided in the inverted pipe (68) and/or in the stirring-mechanism support (70).

15. Biogas plant according to Claim 14, **characterized in that** the inverted pipe (68) and therefore the submersible motorized stirrer (67) can be raised or lowered with a hoisting apparatus (73) which can be arranged outside the fermentation vessel, preferably with a positionally fixed cable winch, the cable (74) of which is guided via a positionally fixed deflection point, preferably a deflection pulley (75), arranged approximately at least at maximum hoisting height of the inverted pipe, to a cable-joining point (76) arranged at the upper end of the inverted pipe.

16. Biogas plant according to Claim 14 or Claim 15, **characterized in that** the inverted pipe (68) has a closed profile cross section, and the stirring-mechanism support has an open profile cross section which is orientated towards the submersible motor.

17. Biogas plant according to one of Claims 1 to 16, **characterized in that** a stirring-mechanism opening (6; 80) is contained above the submersible motorized stirrer (16) in the fermentation-vessel top wall (5), **in that** a service dome (7) is fitted in a gastight manner over the stirring-mechanism opening (6) and the stirring-mechanism support (10; 58; 64) protrudes with its upper end into the region of the said service dome and the submersible motorized stirrer (16; 67) can be hoisted into it, and **in that** at least one power line (20; 45; 51; 56; 62; 77) is introduced in a gastight manner into the fermentation vessel (2) through a service-dome wall.

## Revendications

1. Installation de biogaz pour la fermentation de matériaux organiques
avec au moins un récipient fermenteur avec une zone liquide inférieure et une zone gazeuse au-dessus de celle-ci,
avec au moins un appareil d'agitation à moteur submersible disposé dans le récipient fermenteur,
avec une conduite d'énergie, qui est conduite de manière étanche, dans une paroi du récipient et dans la zone gazeuse, pour le moteur submersible de l'appareil d'agitation à moteur submersible,
**caractérisée en ce que** le moteur submersible est un moteur pouvant fonctionner par un milieu de travail,
**en ce que** la conduite d'énergie (20 ; 45 ; 51 ; 56 ; 62 ; 77) d'une part, comprend une conduite tubulaire ou de type tuyau comme conduite d'alimentation (18 ; 55) pour le milieu, qui est raccordée à l'unité de compression du milieu (34), et d'autre part, comprend une conduite tubulaire ou de type tuyau comme conduite d'enlèvement (19 ; 54) pour le milieu,
**en ce que** la conduite d'enlèvement (19,54) part du moteur submersible par la zone gazeuse (4) et de manière étanche, par une paroi du récipient depuis le récipient du fermenteur (2), et
**en ce que** l'unité de compression du milieu (34) peut être mis en fonctionnement de manière électrique et est disposé à l'extérieur du récipient fermenteur (2).

2. Installation de biogaz selon la revendication 1, **caractérisé en ce que** le moteur submersible est un moteur hydraulique, où le milieu est un liquide hydraulique et l'unité de compression (34) est un compresseur hydraulique, et
**en ce que** la conduite d'enlèvement (19,54) est raccordée, comme une conduite de recyclage dans un cycle hydraulique fermé, au compresseur hydraulique (34).

3. Installation de biogaz selon la revendication 1, **caractérisé en ce que** le moteur submersible est un moteur pneumatique, où le milieu est l'air et l'unité de compression (34) est un compresseur à air, et
**en ce que** la conduite d'enlèvement (19,54) est conduite comme une conduite de purge, de manière étanche depuis le récipient fermenteur (2).

4. Installation de biogaz selon l'une quelconque des revendications 1 à 3, **caractérisé en ce que** d'autres moteurs hydrauliques et/ou moteurs pneumatiques sont utilisés comme moteurs de pompe et/ou moteurs à appareil inférieur et/ou moteurs de dispositif protecteur et/ou moteurs à chemise tiroir sur les conduites tubulaires et/ou moteurs de levage (30) et/ou moteurs oscillants (27), et **en ce que** tous les moteurs peuvent être mis en fonctionnement depuis une unité centrale de compression (34) par une unité de contrôle (33).

5. Installation de biogaz selon l'une quelconque des revendications 1 à 4, **caractérisé en ce que** l'appareil d'agitation à moteur submersible (16) est disposé de manière à être verticalement ajusté par un dispositif de levage,
**en ce que** le dispositif de levage comprend un support d'agitateur (10 ; 58 ; 64) en forme de montant, installé verticalement dans le récipient, sur lequel l'appareil d'agitation à moteur submersible (16) est maintenu de manière mobile, et
**en ce que** dans la partie inférieure du support d'agitateur (10 ; 58 ; 64), est disposé comme constituant d'un treuil à câble manoeuvrable, un tambour à câble (13) avec une liaison des câbles par un câble enroulable (15 ; 47 ; 52) à l'appareil d'agitation à moteur submersible (16).

6. Installation de biogaz selon la revendication 5, **caractérisé en ce que** la conduite d'énergie (45) est disposée dans la partie supérieure du support d'agitateur (10), et
**en ce que** la conduite d'énergie (45) en tuyau libre (46) est enroulé autour du câble (47), où les tuyaux (46) sont plus ou moins écrasés selon la position ajustable de l'appareil d'agitation à moteur submersible (16).

7. Installation de biogaz selon la revendication 5, **caractérisé en ce que** pour la conduite d'énergie (51), on prévoit un tambour manoeuvrable comme tambour de la conduite d'énergie (50), sur lequel la conduite d'énergie (51) peut être enroulée, de manière correspondante et coordonnée à l'état d'enroulement du câble, et
**en ce que** le tambour de la conduite d'énergie (50) présente un joint tournant axial (53), duquel, d'une part, une partie de la conduite d'énergie (51) longe une traverse stationnaire de la paroi du récipient et d'autre part, une partie de la conduite d'énergie (51) peut être enroulée et déroulée sur le tambour de la conduite d'énergie (50).

8. Installation de biogaz selon la revendication 7, **caractérisé en ce que** le tambour de câble (13) et le tambour de la conduite d'énergie (50) ont le même axe et sont couplés par transmission de mouvement rotatif

9. Installation de biogaz selon la revendication 7, **caractérisé en ce que** le tambour de câble (13) est identique au tambour de la conduite d'énergie, et
**en ce que** la conduite d'énergie (20) et le câble (15) sont reliés sur leur longueur.

10. Installation de biogaz selon la revendication 9, **caractérisé en ce que** le câble (15) est formé d'un treillis en acier (17), qui entoure la conduite d'alimentation (18) et/ou la conduite d'enlèvement (19) y intégrées, de la conduite d'énergie (20).

11. Installation de biogaz selon l'une quelconque des revendications 5 à 10, **caractérisé en ce que** le câble et/ou la conduite d'énergie (56, 62) sont conduits dans un tube télescopique (57, 63) consistant en plusieurs tubes télescopiques , et
**en ce que** le tube télescopique (57, 63) s'étire verticalement le long du support d'agitateur (58, 64), où le tube télescopique supérieur (60, 65) est disposé dans une zone supérieure du récipient fermenteur (2) et **en ce que** le tube télescopique inférieur (61, 66) est relié directement ou indirectement à l'appareil d'agitation à moteur submersible (16) de sorte que le tube télescopique (57, 63) est extrait en une position inférieure de l'appareil d'agitation à moteur submersible (16) et rassemblé en une position supérieure.

12. Installation de biogaz selon la revendication 11, **caractérisé en ce que** le tube télescopique (57) est disposé dans le support d'agitateur (58) et **en ce que** le support d'agitateur (58) présente une fente longitudinale (59), par laquelle on dispose la liaison à l'appareil d'agitation à moteur submersible (16).

13. Installation de biogaz selon la revendication 11, **caractérisé en ce que** le tube téléscopique (63) entoure l'agitateur (64) et **en ce que** le câble et/ou la conduite d'énergie (62) est conduite entre le support d'agitateur (64) et la paroi interne du tube télescopique.

14. Installation de biogaz selon l'une quelconque des revendications 1 à 4, **caractérisée en ce que** l'appareil d'agitation à moteur submersible (67) est disposé sur un tube à recouvrement (68), de préférence sur une extrémité inférieure d'un tube à recouvrement, qui est conduit de manière mobile dans le récipient fermenteur, de manière télescopique, de préférence avec une distance de séparation (69), avec un support d'agitateur (70) en forme de montant, disposé verticalement dans le récipient, pouvant être enfoncé et déplacé en direction longitudinale du support d'agitateur pour ajuster différentes positions en hauteur du moteur submersible,
**en ce que** le tube à recouvrement (68), en chaque position en hauteur du moteur submersible, traverse le récipient fermeteur, de manière étanche pour au moins une zone partielle du tube à recouvrement (68), et
**en ce que** la conduite d'énergie (77) est conduite dans le tube à recouvrement (68) et/ou le support d'agitateur (70).

15. Installation de biogaz selon la revendication 14, **caractérisée en ce que** le tube à recouvrement (68), et ainsi l'appareil d'agitation à moteur submersible (67) peuvent être montés et descendus avec un dispositif de levage (73) pouvant être disposé à l'extérieur du récipient fermenteur, de préférence avec un treuil à câble fixe, où le câble (74) passe par un point de retournement fixe, disposé sur le tube creux du tube à recouvrement maximal, de préférence une poulie de guidage (75), vers un site de liaison du câble (76) disposé sur l'extrémité supérieure du tube à recouvrement.

16. Installation de biogaz selon la revendication 14 ou 15, **caractérisée en ce que** le tube à recouvrement (68) présente une coupe transversale fermée et le support d'agitateur, une coupe transversale ouverte ajustée au moteur submersible.

17. Installation de biogaz selon l'une quelconque des revendications 1 à 16, **caractérisée en ce que** au-dessus de l'appareil d'agitation à moteur submersible (16), on trouve une ouverture d'agitateur (6, 80) dans le couvercle du récipient fermenteur (5),
**en ce que** par l'ouverture d'agitateur (6), un dôme de service (7) est monté de manière étanche aux gaz, dans la zone duquel le support d'agitateur (10, 58, 64) dépasse par son extrémité supérieure et dans lequel l'appareil d'agitation à moteur submersible (16, 67) peut être extrait, et
**en ce qu'**au moins une conduite d'énergie (20, 45, 51, 56, 62, 77) est introduite de manière étanche aux gaz, par une paroi du dôme de service, dans le récipient fermenteur (2).
